Europäisches Patentamt

European Patent Office

Office européen des brevets

(19)

(11) **EP 1 136 914 A2**

(12) **EUROPEAN PATENT APPLICATION**

(43) Date of publication:
26.09.2001 Bulletin 2001/39

(51) Int Cl.⁷: **G06F 17/00**, G06F 19/00

(21) Application number: 00128526.1

(22) Date of filing: 27.12.2000

(84) Designated Contracting States:
AT BE CH CY DE DK ES FI FR GB GR IE IT LI LU MC NL PT SE TR
Designated Extension States:
AL LT LV MK RO SI

(30) Priority: 24.03.2000 IT BO200166

(71) Applicant: **UNIVERSITA DEGLI STUDI DI BOLOGNA**
40126 Bologna (IT)

(72) Inventors:
• **Campanini, Renato**
46030 San Giorgio di Mantova (IT)
• **Bazzani, Armando**
40127 Bologna (IT)

• **Bevilacqua, Alessandro**
40124 Bologna (IT)
• **Brancaccio, Rosa**
63014 Montegranaro (IT)
• **Lanconelli, Nico**
48010 Fusignano (IT)
• **Riccardi, Alessandro**
43030 Parola di Fontanellato (IT)
• **Romani, Davide**
47838 Riccione (IT)

(74) Representative: **Jorio, Paolo et al**
STUDIO TORTA S.r.l.,
Via Viotti, 9
10121 Torino (IT)

(54) **Method and apparatus for the automatic detection of microcalcifications in digital signals of mammary tissue**

(57)    Method for the automatic detection of microcalcifications in a digital signal representing at least one image of at least one portion of mammary tissue; method comprising the following phases:

• detecting at least one potential microcalcification in the digital signal;
• calculating a set of characteristics for the potential microcalcification; and finally
• eliminating, or maintining, the potential microcalcification, using a classifier known as a Support Vector Machine (SVM), on the basis of the characteristics calculated.

Figure 1

EP 1 136 914 A2

**Description**

The present invention refers to a method and an apparatus for the analysis, processing and automatic detection of microcalcifications in digital signals of mammary tissue.

In Europe and the United States, breast cancer is absolutely the most widespread form of neoplasia among women and is also one of the main causes of mortality of the female population.

Mammography is a direct radiological examination of the breast which allows the display of all its anatomic components, showing up any pathological alterations. A beam of X rays passes through the breast and the different absorption capacity of the tissues encountered is recorded on a radiation-sensitive plate.

The discovery of mammography brought a real revolution in the fight against breast cancer.

Thanks to the unceasing technological development and to the refining of the method, modern mammography is able to display lesions of a few millimetres in completely asymptomatic women, allowing a significant advance in diagnosis which is fundamental for an early diagnosis.

On a mammography plate, very bright areas are associated with the glandular tissue and the milk ducts (high power of radiation absorption, radioopaque areas), while the fatty tissue, concentrated in the outer part of the breast, is much darker (low power of X ray absorption, radiolucent area). The anomalies due to present or developing pathologies have different radiation absorption characteristics from those of healthy tissue, so they are shown up in the mammographic examination.

One of the most significant anomalies is microcalcification. It appears typically as a tiny bright mark with a clear edge; its dimensions range in diameter from 0.1 to 1 mm and it assumes considerable clinical importance if clusters of at least five are found in an area of 1 cm x 1cm. The detection of clusters of microcalcifications is the principal aid for the early diagnosis of breast tumours. Generally the structure of the mammary tissue generates a very noisy background, making it difficult to detect these signals.

The advent of new digital technologies allowed computerised analysis of the mammograms. Since then, different computerised systems (CAD, Computer Assisted Diagnosis) have been conceived in order to assist the radiologist in his diagnosis. A CAD system processes a mammographic image and identifies any suspicious areas to be subjected to examination by the radiologist (prompting). To be of assistance in the early diagnosis of mammary carcinoma, the computerised system must be able to detect clusters of microcalcifications. It must be very sensitive, so as to find the microcalcifications that the radiologist could not see; in this way it may replace a second radiologist, allowing a reduction of both the times and costs of a diagnosis.

It is equally important that the system should not highlight areas with signals of another nature (false-positives), as this would increase the time necessary for diagnosis and reduce the specialist's trust in the use of such a solution.

Two different types of error may be made during the reading of a mammogram: errors due to false-positives and errors due to false-negatives.

A *false-negative error* occurs when a mammogram containing any type of lesion is erroneously classified as normal. In other words, the lesion is not detected by the doctor and the woman who presents symptoms of breast carcinoma is diagnosed as healthy. This type of error is clearly the more serious, because a delay in the diagnosis and treatment of the condition may irremediably damage the woman's health.

The second type of error, known as a *false-positive error*, is made when, in a normal mammogram, lesions are indicated which do not in fact exist. Although this type of error does not influence the patient's probabilities of survival, it may produce negative psychological consequences in the woman.

In fact, any diagnosis of breast tumour following a mammographic examination produces in the patient great anxiety about her state of health.

In a mammogram, the lesions may appear with a great variety of forms, dimensions and level of contrast. Similarly, the density and complexity of the mammary tissue which forms the structured background of the image may assume notable variations. It may therefore occur that a cluster of microcalcifications is particularly clear and easy to detect in a certain area of the mammogram, while in areas where the contrast between the calcifications and the background is low its detection may require an attentive and systematic analysis of the image. This suggests the accuracy of a radiologist's work may benefit if the doctor's attention is directed, by means of an automatic system, towards those areas of the image in which suspicious lesions are present.

In this type of identification processes, the use of automatic classifiers is known, for example neural networks, which comprise a training phase. Generally, a classifier is developed considering only the "empirical risk functional" that it makes in this phase, without considering its behaviour in the presence of a signal that has never been analysed.

The present invention, on the other hand, aims to overcome the above-mentioned disadvantage, providing an innovative process, and the respective method, which uses a classifier based on the Statistical Learning Theory called Support Vector Machine (SVM). During the learning phase, this classifier considers not only the already mentioned "empirical risk functional", but also a term, called "confidence interval", which depends on the classifying capacity of the classifier itself and on the number of the training examples. The sum of the "empirical risk functional" and of the "confidence interval" provides an upper limit of the so-called "risk functional", or "generalisation error", which gives a precise indication of the real performance of the classifier. In the present invention, the above-mentioned SVM classifier is used in the false-positive reduction phase; this step is of fundamental importance as it allows the false signals revealed by the automatic method to be separated from true microcalcifications.

Although in the continuation of the present description we shall refer expressly to a mammogram, it remains understood that the teachings of the present invention may be applied, making the necessary changes, to the analysis and processing of digital signals of portions of mammary tissue received with any method of investigation and detection, such as, for example, Nuclear Magnetic Resonance, thermography, ultrasonography, scintimammography, CT, PET, etc.

The principal aim of the present invention is therefore to provide a method for the automatic detection of microcalcifications in a digital signal representing at least one portion of mammary tissue; method comprising the following phases:

- detecting at least one potential microcalcification in said digital signal;
- calculating a set of characteristics for said at least one potential microcalcification; and finally
- eliminating, or maintaining, said at least one potential microcalcification, using a classifier known as a Support Vector Machine (SVM), on the basis of the characteristics calculated.

Another aim of the present invention is a method for storing the information on areas of interest present in said digital signals, using a screen table.

Another aim of the present invention is a method for classifying the areas of interest of a digital mammographic image according to their degree of malignity.

A further aim of the present invention is a physical apparatus for implementing the above-mentioned methods.

The present invention shall now be described with reference to the enclosed drawings, which illustrate examples of embodiment without limitation; in which:

- figure 1 is a flow diagram illustrating a first embodiment of an automatic detection method, a method to which the present invention refers;
- figure 2 is a flow diagram illustrating a second embodiment of an automatic detection method, a method to which the present invention refers;
- figure 3 is a histogram of a 12-bit digitised mammographic image;
- figure 4 is a flow diagram illustrating an algorithm for autocropping of the digital image;
- figure 5 shows a flow diagram of a first method of detection used in the systems represented in figures 1, 2;
- figure 6 illustrates a distribution of the standard deviation of the local contrast for a digital image;
- figure 7 shows the standard deviation of the local contrast and the noise level for a digital image after the noise equalising procedure;
- figure 8 shows a matrix representing the coefficients of a first filter;
- figure 9 shows a matrix representing the coefficients of a second filter;
- figure 10 shows the histograms of two different regions of the filtered image; where (a) refers to an area without microcalcifications, and (b) refers to an area containing microcalcifications, and (c) illustrates the details of the tail of (b);
- figure 11 illustrates an example of correction of the background of a region of interest (ROI);
- figure 12 shows the characteristics calculated in the false-positive reduction phase;
- figure 13 illustrates the trend of errors as a function of the dimension VC;
- figure 14 shows a flow diagram of the "boot-strap" learning strategy;
- figure 15 shows a flow diagram of a second method of detection used in the systems represented in figures 1, 2;
- figure 16 schematically illustrates the Fast Wavelet Transform (FWT) method;
- figure 17 schematically illustrates a flow diagram of the wavelet filter;
- figure 18 shows the distribution of the grey levels in regions without microcalcifications (a, b), and in regions with microcalcifications (c, d);
- figure 19 illustrates an example of brightness distribution inside a window and fitting with a parabolic type curve;
- figure 20 shows the forms used for cleaning the window;
- figure 21 illustrates the possible replies of an observer in a simple decision-making pattern of the "Yes/No" type;
- figure 22 shows an example of Free-Response Operating Characteristic (FROC) ;
- figure 23 shows a flow diagram of the classification phase of the ROI according to their degree of malignity;
- figure 24 shows a procedure used for eliminating the structured background in the ROI; and finally
- figure 25 illustrates a flow diagram of the parameters optimisation phase with a genetic algorithm.

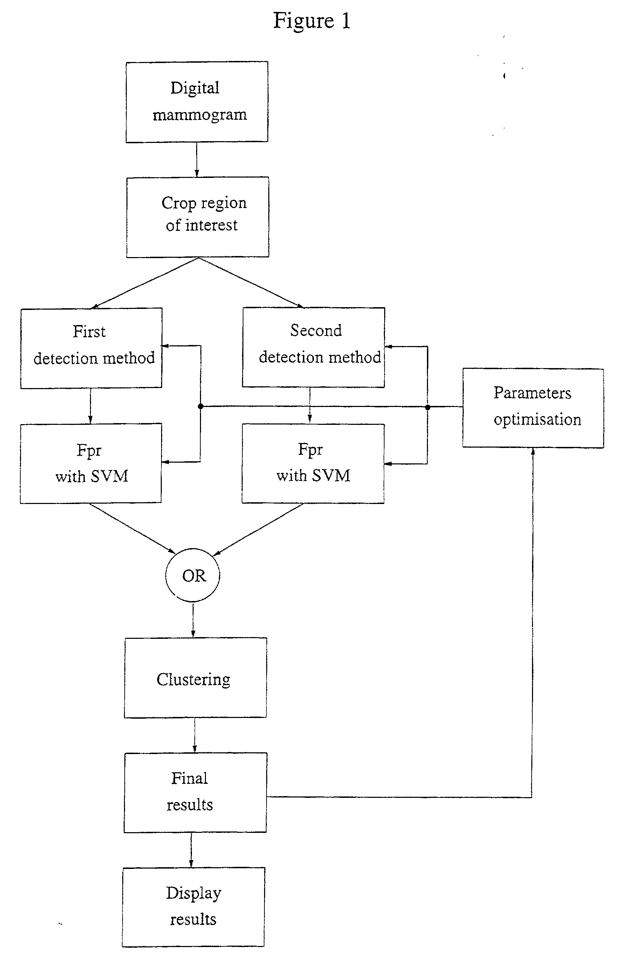

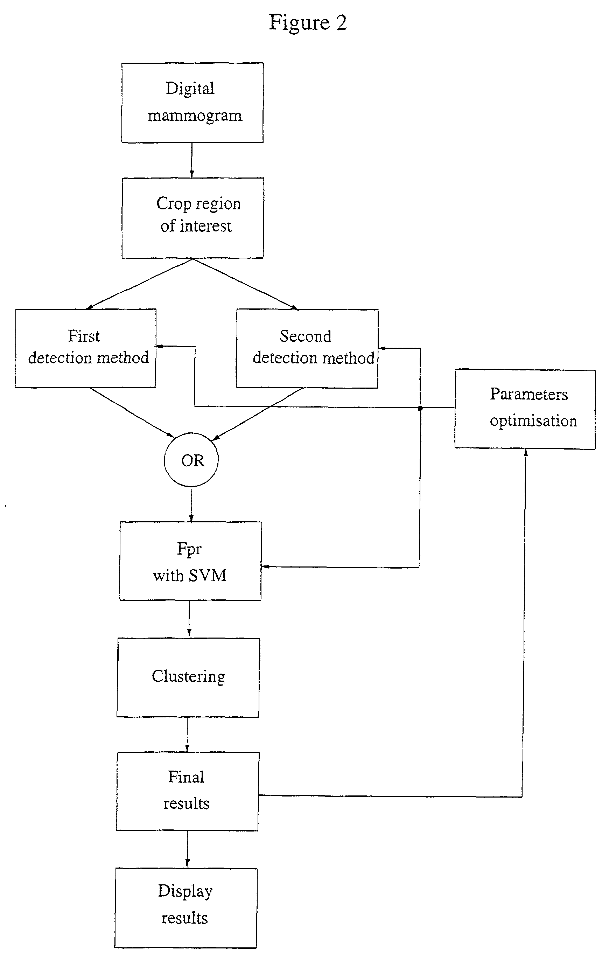

The first step in the method for the automatic detection of clusters of microcalcifications represented in figure 1 and figure 2 is the acquisition of the digitised image.

This process is carried out with a digital mammograph or using CCD or laser scanners.

This is followed by an autocropping phase in which one tries to eliminate from the digital image everything that does not include the mammary tissue.

This image is then passed on to the two detection methods.

In a first embodiment of the method (figure 1), after autocropping of the image a false-positive reduction phase (fpr), based on the use of a SVM classifier, is carried out separately in each of the methods. The signals coming from the classifier are linked by the logic operation OR.

In a second embodiment (figure 2) the signals detected by the two methods are first linked by the logic operation OR, then passed on to the SVM classifier.

The signals which pass the *fpr* phase are then regathered in groups (clustering phase).

Lastly, the final results are shown on the monitor, for example by means of coloured circumferences highlighting the interesting areas detected by the method.

The choice of the parameters involved in the detection and SVM classification phases is optimised thanks to the use of a genetic algorithm.

Going into greater detail, it may be said that the digital mammograms may be obtained in two distinct ways, a primary way and a secondary way. The primary method allows digital mammograms to be obtained directly by recording the transmitted beam of X rays in digital form. This technique does not therefore contemplate the use of the conventional radiographic film. With the secondary method, the radiographic image is first recorded on film and is digitised only later by means of a suitable scanner or CCD camera.

The digital images of the method here described come from the secondary method and have a depth of 12 bit (4096 grey levels) and a space resolution of 100 $\mu$m.

As has already been said, the first operation to be carried out on the image consists of recognising the area occupied by the mammary parenchyma.

In the present invention, the recognition of the area occupied by the breast is obtained from an analysis of the histogram of the image.

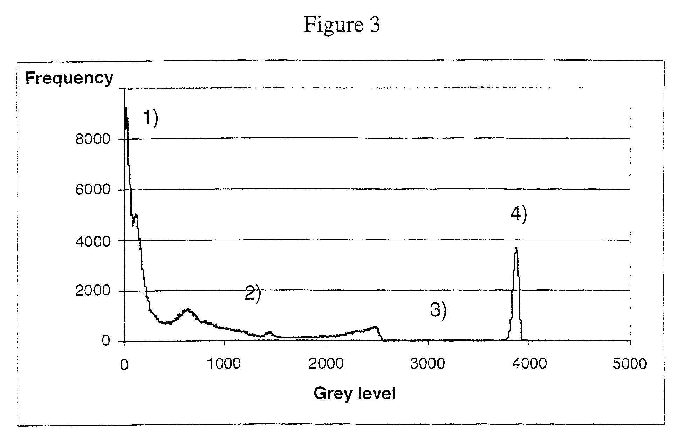

Mammographic images are suitable for this type of approach, since their histogram (figure 3) systematically presents the following characteristics:

- a peak in the darkest region, corresponding to the surface of the film exposed directly to the X rays;
- a long tail corresponding to the mammary tissue;
- a wide interval with almost zero frequency;
- a possible peak in the lightest region, corresponding to regions through which the X rays did not cross, writing and markers, areas acquired by the scanner outside the radiographic plate.

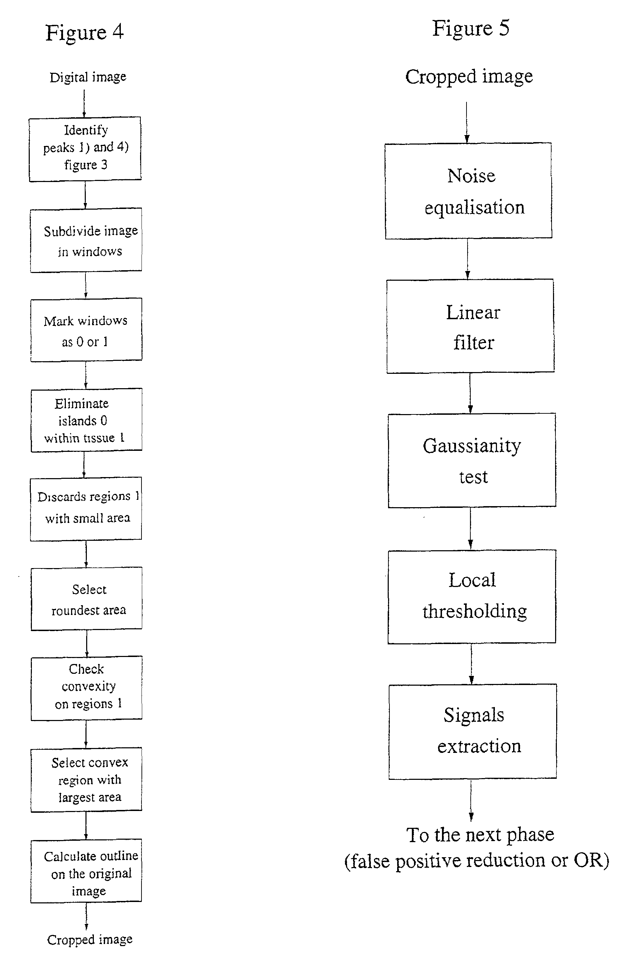

    The autocropping algorithm performs the operations schematically represented in the flow diagram in figure 4.

The first method of detection is represented in the flow diagram shown in figure 5.

In this figure 5 it is possible to distinguish a first step which refers to the noise equalisation.

The basic idea of this noise equalisation is to make the noise itself independent of the grey level value.

Due to the physical properties of the image formation process, the information which it contains presents statistical errors to which the name of noise is given. Although the radiographic images have high contrast and high space resolution, the identification of details of the image becomes difficult when the noise level is high with respect to the details that are important from the diagnostic point of view.

The noise is not uniformly distributed in the image, but depends on the attenuating properties of the tissue represented. In other words, the noise level is considerably higher in the brightest regions of the radiography, which represent dense tissue. Characteristics taken from different regions of the image therefore present different statistical variations. To detect, with the same probability, objects situated in different regions of the image, the algorithm which extracts their characteristics must take into account dependence on the grey level noise. Equalisation may be seen as a non linear transformation of the grey levels which leads to obtaining a constant noise level in each region of the image. In this way, the characteristics extracted by the automatic method present the same statistical deviations, and the signals may be detected irrespective of the considered region of the image.

The steps to perform noise equalisation are the following:

- calculation of the local contrast;
- estimate of the standard deviation of the local contrast;
- calculation of the transformation to be applied to the image.

To calculate the local contrast $c_p$, the following formula was used

$$c_p = I(p) - \frac{1}{N} \sum_{q \in \partial_p} I(q)$$

where $I(p)$ is the grey level in point p, and $\partial_p$ a neighbourhood of the point p composed of N points.

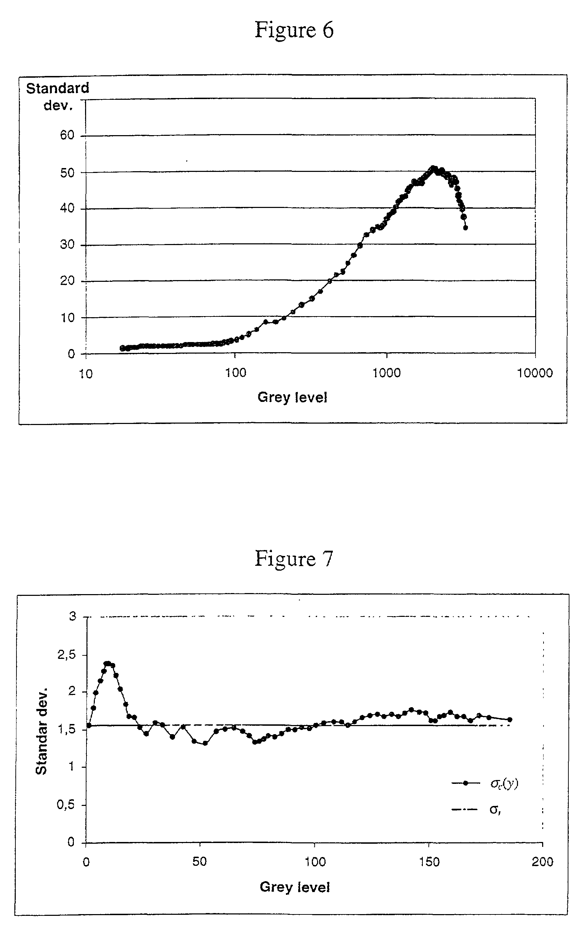

To obtain a reliable value of the standard deviation of the local contrast $\sigma_c(y)$, a high number of points p is necessary such that $I(p)=y$, for each grey level $y$. This requirement is not satisfied for each value of $y$. To overcome this problem, the grey scale is subdivided into a number $K$ of intervals *(bin)*. For each interval $k$ the mean value of the local contrast $c(k)$ is calculated and the standard deviation $\sigma_c(k)$; an interpolation is then carried out on $\sigma_c(k)$ so as to obtain an estimate of $\sigma_c(y)$ for each grey level $y$. Figure 6 shows a typical distribution of $\sigma_c(k)$.

To perform the interpolation on $\sigma_c(k)$, an interpolation with a third degree polynomial was used.

The known transformation used is the following:

$$L(y) = \sigma_r \cdot \int^y {}_0 \frac{1}{\sigma(t)} dt$$

where $\sigma_r$ is the constant level of the standard deviation of the local contrast of the transformed image.

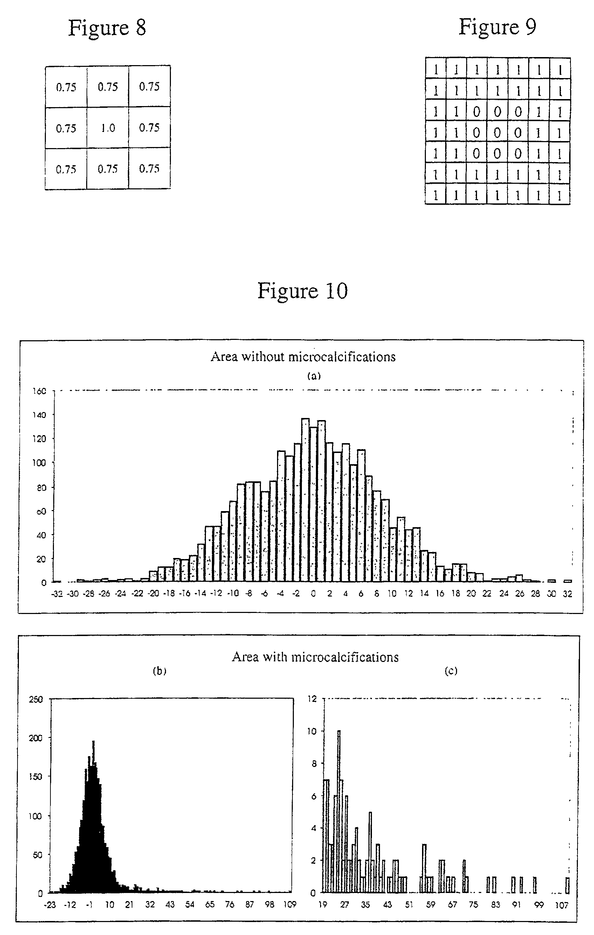

Applying the transformation $y \rightarrow L(y)$ to the grey level of each point, an image is obtained in which the noise $\sigma_c$ is more or less independent of the grey level considered. Figure 7 shows the trend of $\sigma_c(y)$ up to a grey level of 200 after the noise equalisation step; note that the only interval of grey levels in which $\sigma_c(y)$ differs appreciable from $\sigma_r$ is the area with low grey levels, of low interest for the recognition of microcalcifications.

In other embodiments of the method to which the present invention refers, the above-mentioned noise equalisation phase is not contemplated. In this case the cropped image is passed directly to the subsequent phases of the detection algorithm.

Considering figure 5 again, we can see that the function of the linear filter is to eliminate, or at least reduce, the contribution of the structured background (low frequency noise). For this purpose a technique known in the field of image processing was used.

In the spatial field the pixel value of the filtered image $x'_{i,j}$ assumes the value:

$$x'_{i,j} = \frac{1}{(2N_1+1)^2} \sum_{n=-N_1}^{N_1} \sum_{m=-N_1}^{N_1} g1_{n,m} \cdot x_{i+n,j+m} - \frac{1}{(2N_2+1)^2} \sum_{n=-N_2}^{N_2} \sum_{m=-N_2}^{N_2} g2_{n,m} \cdot x_{i+n,j+m}$$

where $(2N_1 + 1)$ is the side in pixel of the mask $g1$, $(2N_2+1)$ is the side in pixel of the mask $g2$, and $x_{i,j}$ is the intensity value of the pixel $(i, j)$ of the initial image. The values of the weight coefficients of the masks $g1$ and $g2$, in the case of images with a resolution of 100 μm, are shown, respectively, in figures 8 and 9.

The image thus filtered contains Gaussian noise and the signals with high contrast of small dimensions.

The third step of the flow diagram of the first detection method illustrated in figure 5 is composed of a Gaussianity test.

The idea behind this test springs from the consideration that, in the filtered image, a region containing only background noise will have a different distribution of intensities from that of an area presenting microcalcifications. In fact, on account of their nature, the microcalcifications will be positioned in the tail of the histogram, at higher intensity values.

Besides, the background noise values taken form a healthy area of the filtered image will follow a Gaussian distribution with mean zero. The presence of microcalcifications will make the distribution asymmetrical (figure 10). A parameter that measures the degree of Gaussianity of distribution may thus be used to discriminate between healthy and non healthy regions.

The Gaussianity test applied calculates a local estimate of the first three moments, indicated as $I_1$, $I_2$ and $I_3$, obtained from the filtered image. More precisely:

$$I_1 = \frac{1}{M \times N} \sum_{i=1}^{M} \sum_{j=1}^{N} x_{i,j}$$

$$I_2 = \frac{1}{M \times N} \sum_{i=1}^{M} \sum_{j=1}^{N} x_{i,j}^2$$

$$I_3 = \frac{1}{M \times N} \sum_{i=1}^{M} \sum_{j=1}^{N} x_{i,j}^3$$

where $x_{i,j}$ is the pixel intensity in position (i, j) in the filtered image and $MxN$ is the area of the local window. In the case of Gaussian distributions $I_1$, $I_2$ and $I_3$ converge on the following values for $M, N \to \infty$:

$$I_1 \to \mu$$

$$I_2 \to \sigma^2 + \mu^2$$

$$I_3 \to \mu^3 + 3\sigma^2\mu$$

where $\mu$ and $\sigma^2$ represent the mean value and the variance of the histogram of the local window.

The expression:

$$G(I_1, I_2, I_3) = I_3 - 3I_1(I_2 - I_1^2) - I_1^3$$

Will tend to zero for Gaussian distributions, while values different from zero will indicate non Gaussianity.

The above-mentioned Gaussianity test may be formulated in the following terms:

$$H_0 : G(I_1, I_2, I_3) < T_G$$

$$H_1 : G(I_1, I_2, I_3) \geq T_G$$

Where $T_G$ is a threshold value of the parameter $G$ which allows discrimination between $H_0$ and $H_1$, which correspond respectively to the cases of healthy regions and regions with microcalcifications. In the preferred embodiment, a value of $T_G$ equal to 0.9 was chosen.

In the fourth step illustrated in figure 5, local thresholding on the grey levels was considered.

This thresholding is applied to the filtered image and its purpose is to isolate the microcalcifications from the remaining background noise.

Once the suspicious regions have been identified, characterised by a high value of the Gaussianity index G, the local thresholding operation contemplates a further statistical test carried out only on the pixels of these regions, with the aim of detecting any presence of microcalcifications.

The method to which the present invention refers again works by calculating local statistical parameters for the distribution of the grey levels of the pixels inside a mask centred on a suspicious region. The statistical measures which are calculated are the mean $\mu$ and the standard deviation $\sigma$.

The pixel on which the window is centred is preserved, that is it is considered part of a possible microcalcification, only if its intensity exceeds the mean value $\mu$ of a predetermined number $k$ of times the standard deviation $\sigma$.

As $k$ varies, the method will have a different sensitivity value.

In the fifth step of the block diagram in figure 5, the signals are extracted in order to localise their position.

This operation is made possible using the binary image obtained from the previous local thresholding step.

The contiguous pixels that have survived thresholding are regrouped in a single structure which represents a potential microcalcification. For each of these signals identified the corresponding mass centre is calculated. The result obtained on completion of this phase is composed of a sequence of coordinates which identify the position of the mass centres of the potential microcalcifications found.

The false-positive reduction phase illustrated in figures 1 and 2 consists of separating signals concerning true microcalcifications from those concerning objects other than the microcalcifications.

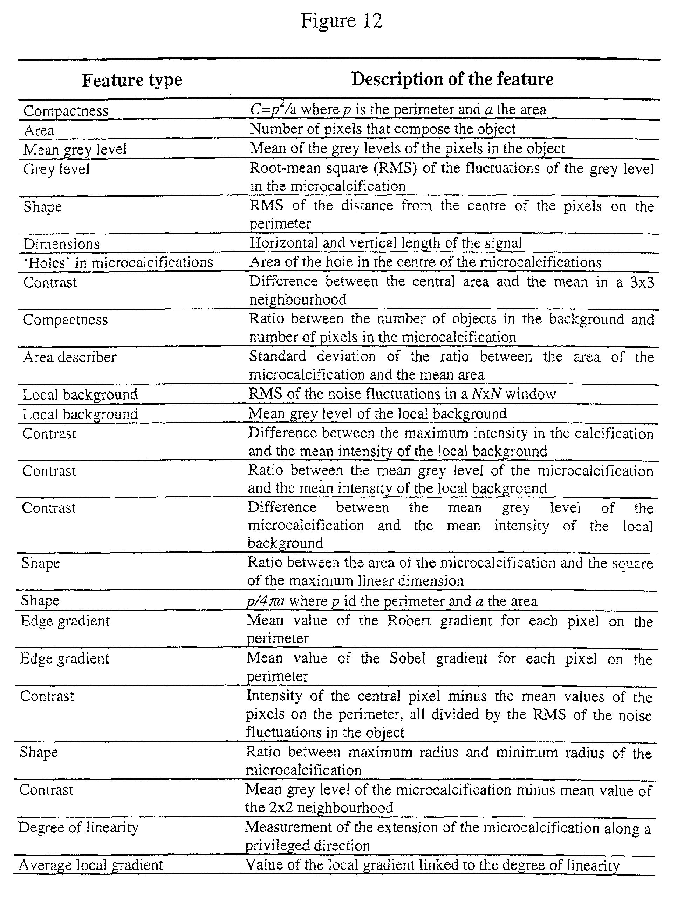

This reduction phase makes use of a series of characteristics which are able to discriminate between true and untrue signals (figure 12).

To determine the value of these characteristics, for a given signal a region of interest (ROI) is extracted from the original digital mammogram; in the preferred embodiment this ROI has a dimension of 32x32 pixel and is centred on the previously identified potential microcalcification. The aim is to isolate the signal from the annoying structured background present in the rest of the ROI. To eliminate, or at least reduce, the influence of the non uniform background, a surface is constructed which approximates the trend of the noise within the ROI.

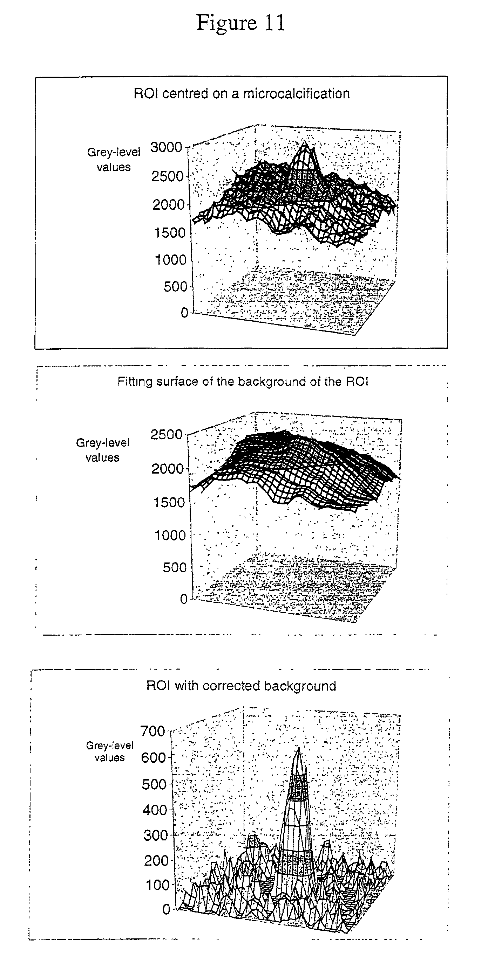

The surface-fitting techniques used is based on polynomial or spline approximation. The surface obtained by means of the fitting process is subtracted from the original ROI, obtaining a new image characterised by a more uniform background. An example of the correction made by the fitting operation is illustrated in figure 11.

It is possible to perform a thresholding operation on the new ROI with a uniform background to isolate the signal and thus determine the pixels of which it is composed. For the signal examined, the characteristics illustrated in figure 12 are calculated. There are 24 of these characteristics in the preferred configuration; clearly it is also possible to use a subset of them or to increase their number.

As has already been said, the discrimination between true signals and false identifications is made by means of a SVM classifier, based on the Statistical Learning Theory.

In other words, in the present invention, the Support Vector Machine is applied in an innovative manner, which in some way improves the traditional CAD systems which, to classify, use methods that are not theoretically justified by the Statistical Learning Theory. The signals revealed by the present method therefore belong either to the class of microcalcifications or to the class of false-positives. The problem of how to separate the microcalcifications from the false-positives consists formally of estimating a function $f(\mathbf{x},\tilde{\alpha}):R^N \rightarrow \{\pm 1\}$, where $f(\mathbf{x},\alpha)$ indicates a family of functions, each one of which is characterised by different values of the vector parameter $\alpha$. The function $f$ has value +1 for the vectors $\mathbf{x}$ of signals belonging to microcalcifications and -1 for $\mathbf{x}$ of false-positive signals. Moreover, $\mathbf{x}$ indicates the vector whose $N$ components are the signal characteristics seen in figure 12. As has been said, the number of these characteristics may be 24 but, generally, it may be any positive integer number.

Learning is realised using input-output training data:

$$(x_1,y_1),...,(x_l,y_l) \in R^N \times \{\pm 1\}$$

The data for training the method to which the invention refers are supplied by radiologists who report areas with clusters of microcalcifications confirmed by biopsy. The learning of the method consists of estimating, using the training data, the function $f$ in such a way that $f$ correctly classifies unseen examples $(x, y)$, that is $f(\mathbf{x},\tilde{\mathbf{a}}) = y$ for examples generated by the same probability distribution $P(x,y)$ as the training data.

If no restrictions are placed on the class of functions from which the estimate $f$ is extracted, there is the risk of not having a good generalisation on signals not used during the learning phase. In fact, for each function $f$ and for each set of tests $(\bar{\mathbf{x}}_1,\bar{\mathbf{y}}_1),...,(\bar{\mathbf{x}}_{\bar{l}},\bar{y}_{\bar{l}}) \in R^N \times \{\pm 1\}$ with $\{\bar{\mathbf{x}}_1,...,\bar{\mathbf{x}}_{\bar{l}}\} \cap \{\mathbf{x}_1,...,\mathbf{x}_l\}=\{\}$, there is another function $f^*$ such that $f^*(\mathbf{x}_i)=f(\mathbf{x}_i)$ for all the $i = 1,..., 1$ but with $f^*(\bar{\mathbf{x}}_i) \neq f(\bar{\mathbf{x}}_i)$ for all the $i = 1, ..., \bar{l}$.

Since only the training data are available, there is no possibility of selecting which of the two functions is preferable. Now it is useful to define the empirical risk functional as:

$$R_{emp}[\alpha] = \frac{1}{l}\sum_{i=1}^{l} L\big(y_i, f(\mathbf{x}_i,\alpha)\big)$$

where $L$ is a general loss function.

The minimisation of the value of the empirical risk functional does not consider the eventual presence of a slight test error. The error in the test phase, taken as the average on all the test examples extracted from the probability distribution $P(\mathbf{x},y)$, is also known as "risk functional" and is defined as:

$$R[\alpha] = \int (y, f(x, \alpha)) \, dP(x, y).$$

The Statistical Learning Theory, or VC (Vapnik Chervonenksis) theory, shows that it is indispensable to restrict the class of functions so that *f* is chosen from a class which has a suitable capacity for the amount of training data available.

The VC theory supplies an upper bound of the risk functional. The minimisation of this bound, which depends both on the empirical risk functional and on the capacity of the class of functions, may be used in the ambit of the principle of Structural Risk Minimisation (SRM). One measurement of the capacity of the class of functions is the VC dimension, which is defined as the maximum number *h* of vectors which can be separated into 2 classes in all $2^h$ possible ways using functions of the class itself. In constructing the classifiers, a bound holds in which, if *h* is the VC dimension of the class of functions that the learning machine can realise and *l* the number of training examples, then for all the functions of that class, with probability at least 1-η, with 0<η≤1, holds the bound:

$$R(\boldsymbol{\alpha}) \leq R_{emp}(\boldsymbol{\alpha}) + \phi\!\left(\frac{h}{l}, \frac{\log(\eta)}{l}\right) = R_G$$

where the confidence term φ is defined as

$$\phi\!\left(\frac{h}{l}, \frac{\log(\eta)}{l}\right) = \sqrt{\frac{h\left(\log\dfrac{2l}{h} + 1\right) - \log\!\left(\dfrac{\eta}{4}\right)}{l}}.$$

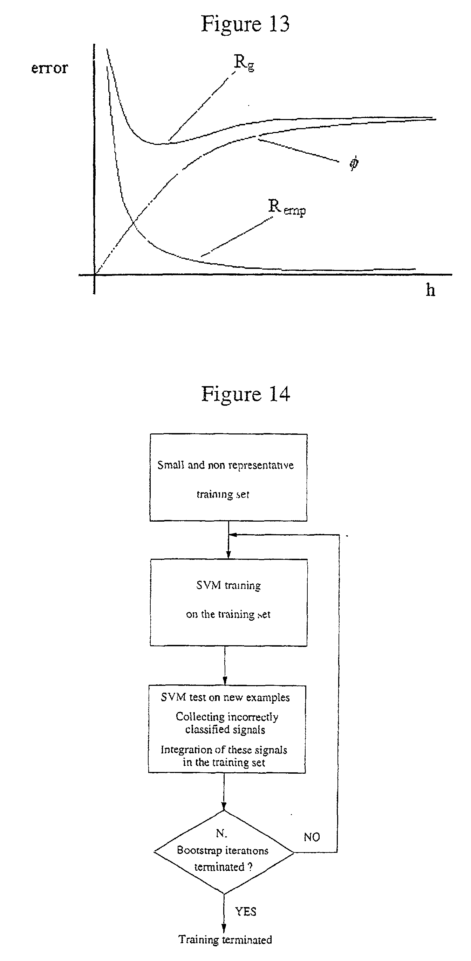

Since the empirical risk functional $R_{emp}$ decreases as h increases while the confidence term φ, for fixed values of *l* and η, grows monotonously with *h* itself (figure 13), classes of functions must be used of which the capacity value may be calculated, so as to be able to assess the value $R_G$. If we consider the class of hyperplanes (**w**·**x**)+*b* = 0 **w** ∈ $R^N$,*b*∈*R*, which corresponds to the decision functions *f*(**x**)=sgn((**w**·**x**)+*b*), to construct f from the experimental data we can use a learning algorithm called *Generalized Portrait,* valid for the separable problems. This algorithm is based on the fact that, among all the hyperplanes that separate the data, there is one and only one which produces the maximum margin of separation between the classes:

$$\max_{\mathbf{w}, b} \min\left\{\|\mathbf{x} - \mathbf{x}_i\| : \mathbf{x} \in R^N, (\mathbf{w} \cdot \mathbf{x}) + b = 0, i = 1, \ldots, l\right\}$$

Maximising the margin of separation coincides with minimising φ*(h,l,*η*)* and therefore $R_G$ once $R_{emp}$ has been fixed, since the relationship

$$h \leq \min([R^2\|w\|^2], N) + 1 = h_{est}$$

holds for the class' of hyperplanes, defined by the normal **w**, which exactly separate the training data belonging to a hypersphere with radius R. To construct the optimal hyperplane we must minimise τ(**w**)=½‖**w**‖² with the constraints $y_i$ · ((**w**·**x**$_i$)+*b*)≥1,*i* =1,...,*l*. This optimisation is treated introducing the Lagrange multipliers $\alpha_i$ ≥ 0 and the Lagrange function:

$$L(\mathbf{w}, b, \boldsymbol{\alpha}) = \frac{1}{2}\|\mathbf{w}\|^2 - \sum_{i=1}^{l} \alpha_i\left(y_i \cdot ((\mathbf{x}_i \cdot \mathbf{w}) + b) - 1\right).$$

This is a problem of constrained optimisation, in which the function to be minimised is a quadratic form, and therefore convex, and the constraints are linear. The theorem of Karush-Kuhn-Tucker may be applied.

In other words, the problem is the equivalent of finding **w**, b, α such that:

$$\frac{\partial}{\partial_w}L(w,b,\alpha) = 0, \ \frac{\partial}{\partial b}L(w,b,\alpha) = 0.$$

with the constraints:

$$\alpha_i \cdot [y_i((x_i \cdot w) + b) - 1] \ 0, i = 1,...,l,$$

$$y_i \cdot ((w \cdot x_i) + b) \geq 1, i = 1,...,l.$$

$$\alpha_i \geq 0$$

This leads to:

$$\sum_{i=1}^{l} \alpha_i y_i = 0$$

and

$$\mathbf{w} = \sum_{i=1}^{l} \alpha_i y_i \mathbf{x}_i.$$

The solution vector has an expansion in terms of a subset of training vectors $\mathbf{x}_i$ of which the $\alpha_i$ are not zero. From the complementary conditions of Karush-Kuhn-Tucker:

$$\alpha_i \cdot [y_i((x_i \cdot w) + b) - 1] = 0, i = 1,...,l,$$

it results that $\alpha_i \neq 0$ only when $y_i((\mathbf{x}_i \cdot \mathbf{w}) + b) - 1 = 0$, that is when the point $\mathbf{x}_i$ belongs to one of the two hyperplanes parallel to the optimal hyperplane and which define the margin of separation. These vectors $\mathbf{x}_i$ are called Support Vectors.
Proceeding with the calculation, the Lagrange function is rewritten considering that $\Sigma^l_{i=1}\alpha_i y_i = 0$ and $\mathbf{w} = \Sigma^l_{i=1}\alpha_i y_i \mathbf{x}_i$ and this gives the expression of the Wolfe dual in the optimisation problem, that is the multipliers $\alpha_i$ are found which maximise

$$W(\boldsymbol{\alpha}) = \sum_{i=1}^{l} \alpha_i - \frac{1}{2} \sum_{i=1}^{l} \alpha_i \alpha_j y_i y_j (\mathbf{x}_i \cdot \mathbf{x}_j)$$

with

$$\alpha_i \geq 0, i = 1,...,l.$$

and

$$\sum_{i=1}^{l} \alpha_i y_i = 0. \quad .$$

with $\alpha_i \geq 0, i = 1,...,l$, and $\Sigma^l_{i=1}\alpha_i y_i = 0$.
The decision function is a hyperplane and it may therefore be written as:

$$f(x) = \mathrm{sgn}\left(\sum_{i=1}^{l} y_i \alpha_i \cdot (\mathbf{x} \cdot \mathbf{x}_i) + b\right);$$

where b is obtained from the complementary conditions of Karush-Kuhn-Tucker.

Generally the set of the microcalcifications and the set of the false-positive signals are not linearly separable in the space of the input vectors **x**. A method is therefore necessary to construct hypersurfaces more general than the hyperplanes. To do this, the data are mapped into another space $F$, called the features space, by means of a non linear mapping $\phi : R^N \to F$, after which the linear algorithm seen previously must be performed in $F$. The construction of the optimal hyperplane in F and the assessment of the corresponding decision function involve only the calculation of scalar products $(\phi(\mathbf{x}) \cdot \phi(\mathbf{y}))$ and never of the mapped patterns $\phi(\mathbf{x})$ in the explicit form. This is of fundamental importance for the objective that we have, since in some cases scalar products may be assessed by means of a simple function (kernel) $k(\mathbf{x},\mathbf{y}) = (\phi(\mathbf{x}) \cdot \phi(\mathbf{y}))$, which does not require the calculation of the single mapping $\phi(\mathbf{x})$. Generally, Mercer's theorem of functional analysis demonstrates that the kernels $k$ of positive integral operators give rise to maps $\phi$ such that holds $k(\mathbf{x},\mathbf{y}) = (\phi(\mathbf{x}) \cdot \phi(\mathbf{y})$. In one embodiment of the invention, polynomial kernels $k(\mathbf{x},\mathbf{y}) = (\mathbf{x} \cdot \mathbf{y} + c)^d$ with $c > 0$ were used. In other embodiments of the invention, sigmoidal kernels $k(\mathbf{x},\mathbf{y}) = \tanh(k(\mathbf{x},\mathbf{y}) + \Theta)$ were used and radial basis functions kernel, such as $k(\mathbf{x},\mathbf{y}) = \exp(-\|x-y\|2/(2\sigma 2))$.

The non linear mapping operation of microcalcifications and of false-positives signals vectors input space in a high dimensionality space is justified by the theorem of Cover on the separability of patterns. That is, a linearly non separable patterns input space may be transformed into a new features space where the patterns are linearly separable with high probability, as long as the transformation is non linear and the dimensionality of the new features space is sufficiently large.

As has been said previously, the SVM finds the optimal separation hyperplane, a hyperplane defined as a linear combination of the new features space vectors and no longer of the input space ones. The hyperplane is constructed in accordance with the principle of Structural Risk Minimisation. In other CAD systems the reduction of false-positives is achieved by means of classification with neural networks. The neural networks minimise the empirical risk functional, which does not guarantee a good generalisation in the application phase.

In the present invention, decision functions with the following form are used for classification:

$$f(\mathbf{x}) = \mathrm{sgn}\left(\sum_{i=1}^{l} y_i \alpha_i \cdot (\phi(\mathbf{x}) \cdot \phi(\mathbf{x}_i)) + b\right) = \mathrm{sgn}\left(\sum_{i=1}^{l} (y_i \alpha_i \cdot k(\mathbf{x},\mathbf{x}_i)) + b\right)$$

For the optimisation problem we pass to the Wolfe dual. In other words, the following function must be maximised:

$$W(\boldsymbol{\alpha}) = \sum_{i=1}^{l} \alpha_i - \frac{1}{2} \sum_{i=1}^{l} \alpha_i \alpha_j y_i y_j k(\mathbf{x}_i, \mathbf{x}_j)$$

with the conditions $\alpha_i \geq 0, i=1,\ldots,l$, and $\Sigma_{i=1}^{l} \alpha_i y_i = 0$.

Often, in practice, there is no hypersurface separating without errors the class of the microcalcifications from the class of the false-positive signals. So there are examples which violate $y_i((\mathbf{w} \cdot \mathbf{x}_i) + b) \geq 1$. To deal with these cases, variables are introduced, called slack variables, $\xi_i \geq 0, i=1,\ldots,l$ with relaxed constraints $y_i((\mathbf{w} \cdot \mathbf{x}_i) + b) \geq (1 - \xi_i), i=1,\ldots,l$. Now it is a question of minimising the new objective function:

$$\tau(\mathbf{w}, \xi) = \frac{1}{2}\|\mathbf{w}\|^2 + C\sum_{i=1}^{l} \xi_i,$$

with the constraints $\xi_i \geq 0, i=1,\ldots,l$ and $y_i((\mathbf{w} \cdot \mathbf{x}_i) + b) \geq 1 - \xi_i, i=1,\ldots,l$, where C is a positive real parameter, to be chosen a priori, while

$$\sum_{i=1}^{l}\xi_i$$

is an upper bound to the total number of errors on the training set. In the case concerned in the present invention, it is opportune to alter the objective function in order to outweigh one class.

We therefore have to minimise the following function:

$$\frac{1}{2}\|\mathbf{w}\|^2 + C^+\sum_{i=1}^{l^+}\xi_i + C^-\sum_{j=1}^{l^-}\gamma_j$$

where $l^+ + l^- = l$, with the conditions $(\mathbf{w}\cdot\mathbf{x}_i)+b\geq 1-\xi_i$ for $y_i=+1$ and $(\mathbf{w}\cdot\mathbf{x}_j)+b\leq -1+\gamma_j$ for $y_j=-1$ with $\xi_i\geq 0, i=1,...,l^+$ and $\gamma_j\geq 0, j=1,..., l^-$, while $C^+$ and $C^-$ are respectively the cost of the false-negatives and of the false-positives errors.

The relative dual problem is therefore that of maximising the following function:

$$L = -\frac{1}{2}\left(\sum_{i,n}\alpha_i\alpha_n k(\mathbf{x}_i\cdot\mathbf{x}_n) + \sum_{j,m}\beta_j\beta_m k(\mathbf{x}_j\cdot\mathbf{x}_m)\right) + \sum_{i,j}\alpha_i\beta_j k(\mathbf{x}_i\cdot\mathbf{x}_j) + \sum_i\alpha_i + \sum_j\beta_j$$

with $0\leq\alpha_i\leq C^+, 0\leq\beta_j\leq C^-, \Sigma_i\alpha_i=\Sigma_j\beta_j$.

It is not known a priori which couple $(C^+,C^-)$ gives the best results. The training is carried out by fixing $C^-$ and varying the ratio $c^+/c_-$ from the value $l^-/l^+$ (in which case $l^-C^-=l^+C^+$) to the value 1. From this variation the points of the FROC (Free Response Receiver Operating Characteristic) curves are obtained. As the ratio $c^+/c_-$ increases the loss of the true microcalcifications is weighed more and more; in this way the sensitivity of the method is increased, reducing its specificity. There are numerous methods for solving the problem of quadratic optimisation. In a preferred embodiment of the present method, the method known as the "Interior Point Method" was used.

The detection of microcalcifications, like most detection problems, is difficult due to the variability of the patterns to be identified. It is also difficult to analytically describe this variability. Through training of the SVM a decision surface is identified. The system thus trained is then used on images which do not contain any microcalcifications or on areas of images which do not contain any microcalcifications. Both the types of regions mentioned may be highlighted using a screen table as described below (see below).

In a preferred embodiment of the present method a training strategy known by the name "boot-strap" is used (figure 14).

At each iteration this procedure adds to the training data the examples incorrectly classified by the SVM. This should improve the performance of the classifier, because it is made gradually more sensitive to the signals which it does not correctly classify. This training strategy is very useful in the case where the classes, or a subset of them, which are to be recognised are not easy to characterise.

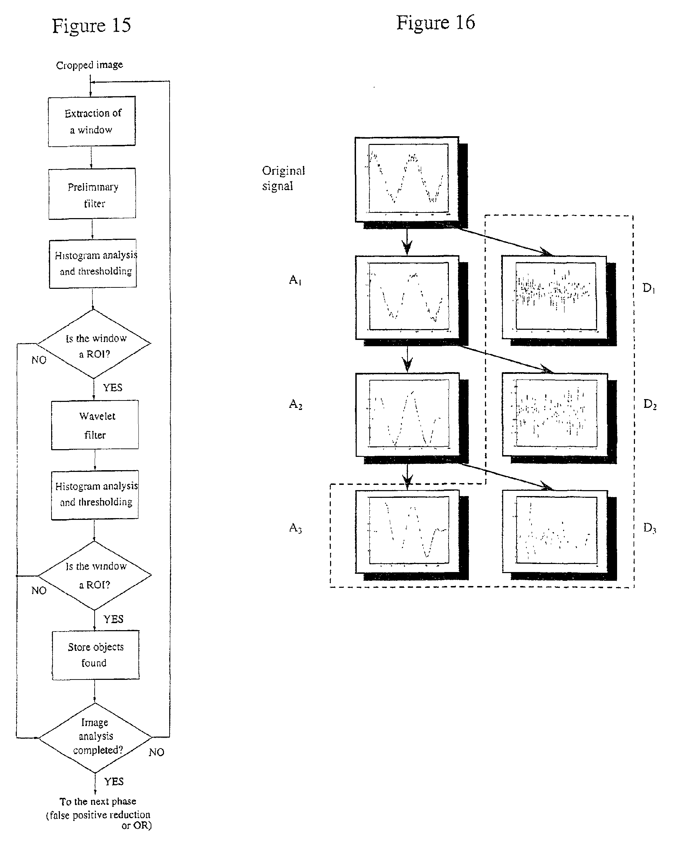

If we refer again to figures 1 and 2, the second detection method follows the general pattern represented in the block diagram in figure 15.

First the search for signals is made, subdividing the mammogram into regions small enough to be able to consider homogeneous the component due to the structure of the mammary tissue. The dimension of the analysis windows was chosen equal to a square of 6x6 mm2 so as to be able to contain at least two microcalcifications. The windows must be partly overlapped, so as to reduce to a minimum the possibility of missing the detection of a group of signals due to incorrect positioning.

Immediately after extraction of the window (figure 15) a preliminary filter is used in order to make the detection phase more efficient. This filter allows identification of the regions in which to apply the wavelet transform. As filter, a linear filter defined as follows was chosen:

$$filt(x, y) = Gauss_n(x,y) - Mean_m(x,y)$$

where $Gauss_n(x,y)$ indicates the result of the convolution of a $nxn$ Gaussian filter at the point $(x,y)$, while $Mean_m(x,y)$ is the average value of the grey levels in a $mxm$ neighbourhood centred on $(x, y)$.

In a preferred embodiment the value of m was set at 9, while the value of $n$ was set at 3.

Still referring to figure 15, the phase concerning the wavelet filter may be analysed in greater detail.

Multiscale analysis by using the wavelet transform transports a function from the spatial domain to another characterised by a family of functions called base functions. They are obtained by translations and dilatations of a single function called mother wavelet $\psi$:

$$\psi_{a,b}(x) = \frac{1}{\sqrt{a}}\psi\left(\frac{x-b}{a}\right), \quad a \in R - \{0\}, b \in R$$

This function must have a mean value of zero and must be localised both in time and in frequency.

An efficient implementation of the discrete wavelet transform is called Fast Wavelet Transform (FWT). The wavelet coefficients are obtained from successive applications of two complementary filters, a high pass one and a low pass one. In the wavelet analysis, with the term "approximation" indicates the large scale components of the signal, while the term "detail" denotes the small scale components.

Figure 16 shows an example illustrating the FWT method.

Initially the two complementary filters described above are applied to the signal, obtaining an approximation $A_1$ and a detail $D_1$ (level 1). In the next step the two filters are applied to $A_1$, obtaining a new approximation $A_2$ and a new detail $D_2$ (level 2). The procedure is repeated, always using the approximation generated in the previous step, until the desired level $n$, obtaining what is called the tree of wavelet decomposition. The greater the level of decomposition, the larger the scale of the relative approximation and of the relative detail, The components enclosed by the broken line in figure 16, that is the approximation $A_n$ and all the details, make up the wavelet decomposition and allow a perfect reconstruction of the initial signal. The procedure for the inverse wavelet transform is the exact opposite of the one just described. That is, it begins with $A_n$ and $D_n$ and generates $A_{n-1}$ using two complementary filters. The procedure continues iterating until the reconstruction of $A_0$, that is of the initial signal. Summing up, it may be stated that the fast wavelet transform generates a multiresolution analysis of a signal, separating it in orthogonal components relating to different spatial scales.

The use of the wavelet transform in the field of detecting signals such as microcalcifications is immediate, as these cover a determined range of scales. It is therefore sufficient to transform the image and to reconstruct it considering only the details relating to the spatial scales concerning the signals to be searched. The scales which contain information on the microcalcifications are the ones with resolutions of 100, 200, 400 and 800 μm.

It emerged that the second and the third scale are the ones which most exalt similar signals, effectively suppressing noise. Scales higher than the third show a high correlation with the structure of the background, while the finest resolution is heavily influenced by the high-frequency noise spread over the whole image. However, completely rejecting the details concerning the first scale makes the identification of some tiny microcalcifications very difficult. Considering this, it was decided to apply hard thresholding to this level of detail. In practice the coefficients relating to the finest detail, which are in modulus less than $k$ times their standard deviation, are cancelled.

To ensure that this system functions efficiently, a mother wavelet is chosen-which is correlated as much as possible with the form of a microcalcification. Symmetrical mother wavelets were used, such as those of the Symlet family (Symmetric Wavelet) and of the LAD family (Least Asymmetric Wavelet), obtaining the best results with the LAD8.

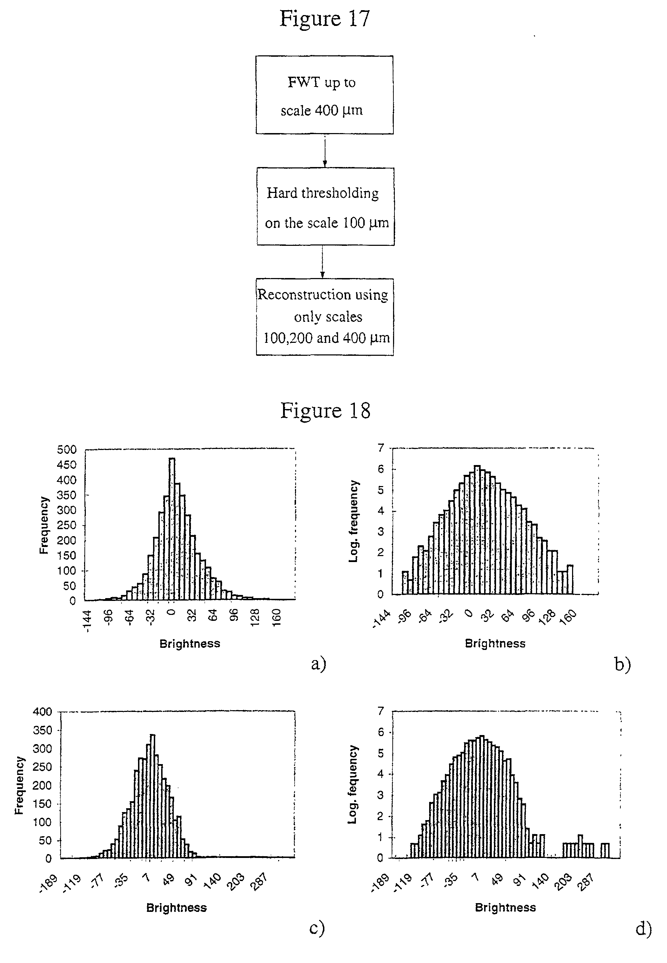

Figure 17 shows the scheme of this wavelet filter.

Returning to figure 15, it may be seen that the step after the filtering stages described above is represented by histogram based thresholding.

After having applied one of the two filters previously described, in this instance a preliminary filter and a wavelet filter, a window is composed solely of signals similar to microcalcifications and noise. It is presumed that the noise has a Gaussian trend. If a window of image without signals is taken, the brightness of its points will be distributed in a Gaussian manner (figure 18 a) while, if a window containing microcalcifications is considered, an anomaly will be seen in the right-hand part of the histogram (figure 18 c). The anomaly are due to the contribution of the pixels belonging to the microcalcifications, which are considerably brighter than the background.

This asymmetry is more evident seen if the histogram is represented on a semilogarithmic diagram (figure 18 b, d).

Considering this last type of graph, a method was obtained for determining the threshold to extract pixels belonging to microcalcifications.

The idea consists of considering the histogram subdivided into two parts, one comprising the grey levels lower than a value $\bar{l}$ and whose trend is due exclusively to Gaussian noise (noise area), the other relative to grey levels higher than $\bar{l}$ and influenced by the presence or absence or microcalcifications (signal area). The search for anomalies is made only in the signal area. In fact, if it contains peaks, the grey level for the first of them will constitute the searched threshold. Clearly if these anomalies do not appear it means that the window does not contain useful signals and so it will be discarded. The problem now shifts to the identification of the value $\bar{l}$.

To have an estimate of $\bar{l}$, the profile of the histogram included in the noise area is approximated with a parabola. $\bar{l}$ is no more than the positive grey level in which the parabola intersects the X axis.

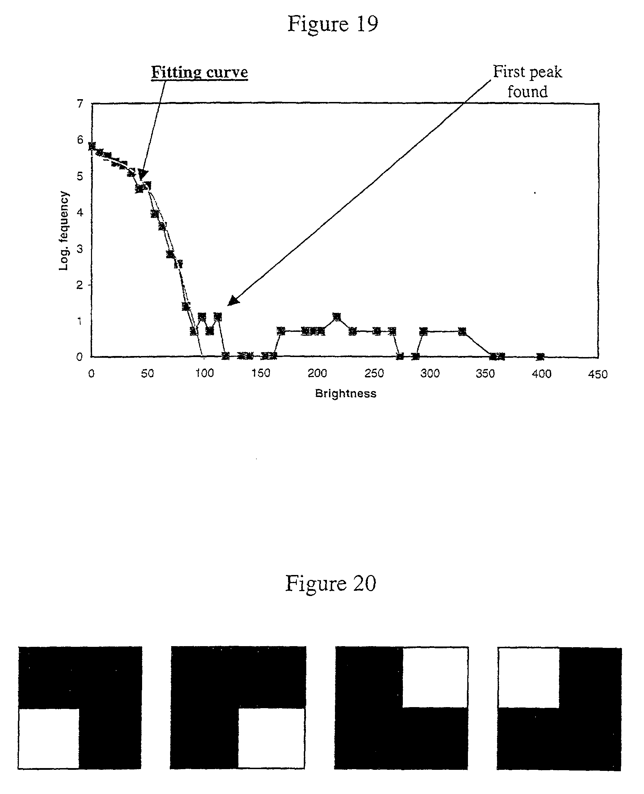

An example of this procedure can be seen in figure 19.

Once thresholding has been applied to the window, the window itself must be cleaned to remove the objects which, for their shape or dimensions, cannot be microcalcifications. This is done by performing a morphological "opening" operation with the four shapes represented in figure 20 and joining the results in a single image through a logic OR. In this way all the structures only one pixel wide are eliminated, leaving the other objects unchanged. The list of the potential microcalcifications is passed on to the false-positive reduction phase described previously.

The 24 characteristics of figure 12 are calculated for each of these signals. At this point, these characteristics are passed directly to a classifier SVM as described previously, following the actuation shape shown in figure 1.

Alternatively, the signals are combined with those detected by the first method by means of an operator OR, following what is illustrated in figure 2.

In the context of the second detection method illustrated in figure 15, a window is considered a "region of interest" (ROI) if, once the thresholding of the histogram has been performed, at least two potential microcalcifications are counted inside it.

Now referring again to figures 1 and 2, we wish to remark that the signals coming from the two detection methods seen above are joined by means of a logic operation OR, giving rise to the global list of the signals which will then be regrouped according to the clustering criterion described below. Since each method is able to report microcalcifications with similar characteristics, by joining together those obtained with different methods it is possible to detect signals with different properties.

The presence of clusters of microcalcifications is in many cases the first and often the only indicator of a breast tumour. In the majority of cases, isolated microcalcifications are not clinically significant. It is for this reason that a CAD for mammography is always oriented towards the search for clusters rather than for single microcalcifications.

The clustering scheme implemented identifies as clusters a group of three or more microcalcifications wherein the distance from the nearest microcalcification is less than 5 mm.

On this point it may be noted that the input data are composed of the list of the coordinates of the mass centres of all the signals identified at the end of the detection phase of single microcalcifications.

For each of the localised signals, the set of those less than 5 mm from each other is determined. If the number in the group is less than three, the signal concerned is eliminated as it is considered isolated, otherwise it survives the clustering phase and goes on to form a group together with the other signals in the set. Once the signals that make up a cluster have been determined, it is characterised by three numbers (*x, y, R*) representing the centre and the radius of the cluster, where *x* and *y* designate the spatial coordinates of the mass centre of the cluster, while *R* represents the distance between the centre of the cluster and the signal farthest away from it.

Apart from what has been said previously, the assessment of the performance of the said method of detection is expressed by the percentage of true clusters found with respect to the number of false-positive clusters generated.

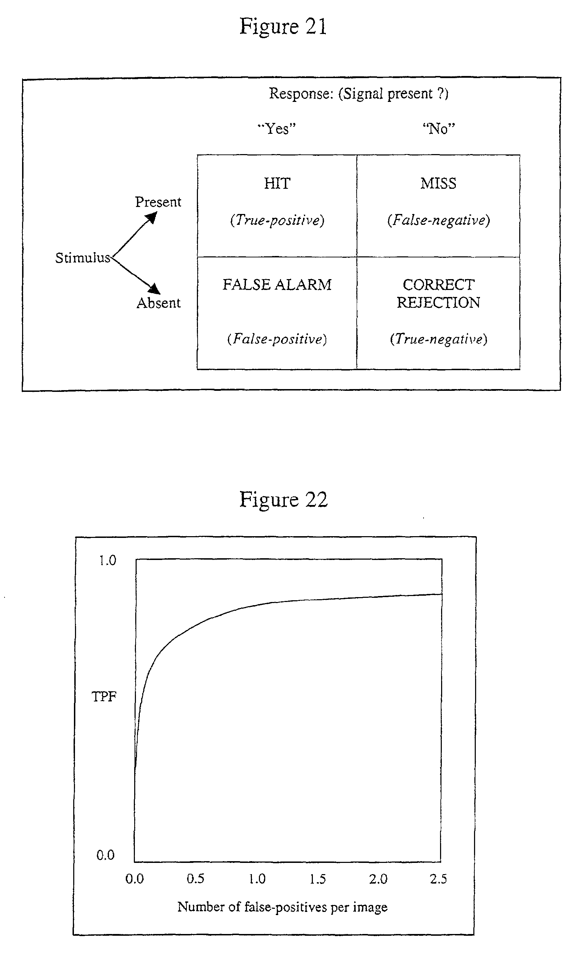

On this point, the detection of a lesion in a radiological image consists of discriminating a signal, represented by the lesion, from a background noise, represented by the normal breast tissue. A simple protocol, for assessing the performances of a radiologist or of an automatic detection method, is represented by the forced discrimination process with two alternatives. According to this scheme, an observer is presented with a series of stimuli, where one stimulus may be only "noise" or "signal + noise". Each time a stimulus is presented, the observer must classify it replying "signal present" or "signal absent". There are then four different possible situations, illustrated in the diagram in figure 21.

The assessment of the performances of an observer, whether this be a doctor or an automatic method, is accomplished in terms of the *Receiver Operating Characteristic* (ROC).

The observer is presented with a sample of radiographic images, some of which contain a single lesion, others are normal, which he must classify. At this point the True-Positive and False-Positive percentages are calculated, as happens in a decision-making process of the "Yes/No" type.

The results produced are illustrated in a graph, giving rise to a ROC curve, constructed with couples of values of the type (P(FP),P(TP)). The values of P(TP) and of P(FP) represent respectively the True-Positive percentages (often indicated as TPF, or *True-Positive Fraction*) and the False-Positive percentages (also indicated as FPF, or *False-Positive Fraction*).

In order to express the performances of a diagnostic method, we introduce indices of "sensitivity", understood as the percentage of images which present a lesion and which are correctly classified, and of "specificity", understood as the percentage of "normal" images classified as such. The True-Positive Fraction and True-Negative Fraction values therefore determine the sensitivity and specificity values of the method.

More precisely:

...

$$[\text{Sensitivity}] = TPF = P(TP)$$

$$[\text{Specificity}] = TNF = 1 - FPF = 1 - P(FP)$$

where TPF, TNF, FPF are respectively the True-Positive, True-Negative and False-Positive Fraction. The performances of a method can therefore be expressed by either "specificity" and "sensitivity", or by FPF and TPF.

Although the ROC analysis can be applied to a vast type of problems of identification and classification of signals, it has one big limit. Its application is limited to those decision-making problems in which the observer, in the presence of a stimulus, is tied to a single reply: "signal present" or "signal absent". In many practical problems this limit is inadequate. Consider, for example, an automatic method for locating an object in a digital image. The algorithm may indicate different points of the image, but only one of these identifies the searched object, while the others are false-positives. Applying ROC analysis, it results that the method has produced a true-positive, because the object has been located. However, the information concerning the false-positives is ignored. To ensure that these data too are included in the analysis, a variation of the ROC curves is used, known as Free-Response Operating Characteristic (FROC). An example of a FROC curve is illustrated in figure 22.

As may be noted, the X axis expresses the number of false-positives per image. There would be no sense in expressing this value as a percentage since, theoretically, there are no limits to the number of false-positives which may be generated.

The FROC curves are the preferred instrument for analysing the performances of an automatic detction method for lesions in digital images.

Once the said assessment of the method's performances has been made, it is possible to display and store the results obtained by the method.

The areas, containing the clusters of microcalcifications indicated by the detection algorithms seen above, are displayed on a screen as coloured circles, with the centre situated in the centre of the cluster and radius equal to the radius of the cluster. These circumferences overlap the original digital image. The information concerning the clusters of an image may therefore be stored in a text file, which is loaded every time anyone wants to display the result of the detection.

The storage of the information concerning the regions of interest, such as, for example, the position and extent of the region itself, may also be carried out by an expert user (radiologist), using the following devices:

- a first device enclosing in a single unit the functions of a screen with liquid crystals (LCD) and a pressure-sensitive graphic table which enables the user, by means of a special pen, to draw directly on the screen surface; this first device may be combined with
- a second device suited for connecting the screen-table to a computer which stores the medical image with the position and the extent of the regions of interest.

    Using these instruments, the doctor can signal, jointly with or as an alternative to the automatic detection method, any regions of interest not signalled by the method. It is also possible for the doctor to decide to signal interesting regions in images not analysed by the method.

The procedure for storing the position and extent of the regions of interest is extremely accurate, though simple.

Firstly, the doctor observes the image in the screen table and marks the outline of the interesting region using a special pen. This information is stored in a text file linked to the image that is being displayed.

Moreover, it is possible to load and display these data along with those concerning the clusters identified by the automatic detection method.

The information on the regions signalled by the doctor may be used both to carry out further training of the automatic detection method and as input data for the method of classifying regions of interest according to their degree of malignity, as described below (see below).

It is possible to train the automatic method again, considering these last regions indicated, if the conditions of the apparatus for acquiring the digital image are modified or if interesting types of signals are presented which were not present in the set of training signals used previously, or in any case in which the user wants to update the training. The method in which training is carried out is the same as the one seen previously.

The ROI of which one wants to know the degree of malignity may come either from the automatic detection method or from the doctor who signals the presence of these regions thanks to the screen table, in the manner just described.

Due too the high heterogeneity of the shapes of microcalcifications and to their being grouped in clusters, there is a certain difficulty in defining the properties which differentiate benign tumours from malignant ones, and which therefore characterise the degree of malignity of the lesions.

Presuming that it is not known a priori which are the best properties for classification according to the degree of malignity the search method with which it was chosen to deal with the problem is of an inductive type.

In fact, all the features definable with this study of the image texture are extracted from each ROI by means of Texture Analysis; only later, observing their distribution in the benign cases and in the malignant cases, those that most differentiate the two classes are selected.

In this phase, selecting the Texture properties is the equivalent of reducing the dimensions of the problem to the intrinsic dimensions rejecting redundant information. For this purpose, classical statistics techniques may be used, such as the Student test and a study of the linear correlation. Finally the selected characteristics will be used as input of a SVM classifier. The performances are measured in terms of "sensitivity" and "specificity", concepts which have already been defined.

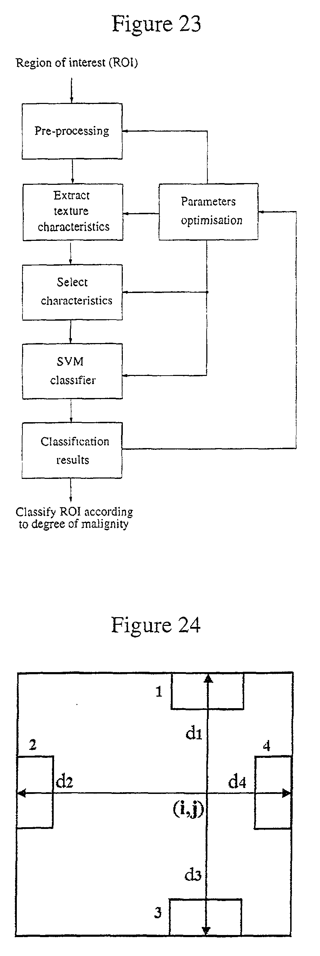

The general scheme of the automatic method is shown in figure 23.

The first step of the procedure illustrated is a preprocessing which allows the structured background to be subtracted from the ROI. Generally, the presence of different tissues is able to influence the composition of the texture matrices and consequently the value of the texture features. To reduce this disturbing factor, it was decided to apply a technique for reducing low-frequency noise.

The procedure implies the calculation of the means of the grey level values of the pixels belonging to the four rectangular boxes on the respective sides of the ROI, as in Figure 24. The estimated grey level value of the structured background $G(i, j)$, of a given pixel $(i, j)$ is calculated as:

$$G(i,j) = \frac{\sum^4_{k=1} g_k / d_k}{\sum^4_{k=1} 1 / d_k}$$

where $g_k$ is the average grey level of the box $k$ at the side of the ROI and $d_k$ is the distance between the pixel and the side $k$ of the ROI. The four boxes are shifted, in the area of the image being processed, along the inside wall of the sides together with the pixel to be estimated. Calculating $G(i, j)$, as a weighted mean along the distances makes the average of the nearest box more influential than that of the farthest away one.

Studying the images available, it may be seen that the ROI have dimensions that may range from 3 to 30 mm. To establish a fixed value for the box dimensions, constant for each ROI, even when they are very small, it is decided to increase the dimension to at least one and a half times the original, always taking a dimension of 15 mm as the minimum limit. In the preferred embodiment it is established to use boxes measuring 6x3 mm$^2$. The image is processed, subtracting the estimated background, that is the new grey level values of the pixel are defined as:

$$I'(i,j)=I(i,j)-G(i,j),$$

where $I'$ is the new grey value of the pixel and $I$ the previous one.

In the ROI thus processed, the information on the microcalcifications has not been modified, but the background has been smoothed, making it less influential.

The second phase of the procedure illustrated in figure 23 concerns the extraction of the texture features.

The intrinsic property of a texture element is well concealed in the image, and the texture may be described by statistics models relative to the analysis of the single pixels. The assumption made is that the texture information of an image is contained entirely in the spatial relationships that the grey levels possess with one another. More specifically it is presumed that the information of the image texture is adequately defined by a set of matrices describing the spatial interrelation of the grey levels calculated at different angles and distances between pairs of contiguous pixels in the image. All the texture characteristics will derive from these matrices commonly called Spatial Grey-Level Dependence (SGLD) Matrices, or even co-occurrence matrices.

To extract the information on the texture properties from the image, we must build the SGLD matrices on the basis of the concept of adjacent or first-neighbour elements. If we consider any pixel in the image, except those on the outside edge of the image, it will have eight neighbouring pixels around it. The pixel examined will therefore have eight first-neighbours which, with respect to it, are arranged along four principal spatial directions $\vartheta$, such as horizontal at $0°$, vertical at $90°$, along the diagonal at $45°$ and finally along the diagonal at $135°$. In this case the first neighbours are being examined, that is the pixels which are separated from each other by only one unit of measurement $d$, but it is also possible to analyse pixels at greater distances, considering the second layer of pixels outside this one, that is the second neighbours, and so on for larger values of $d$.

Assuming that the texture information is correlated to the number of times in which pairs of pixels are arranged in a given reciprocal spatial configuration, the SGLD matrix element $P(i,j|d,\vartheta)$ is defined as the probability which the pair of grey levels $i$ and $j$ have of appearing within the image at a distance d from each other and at an angle of $\vartheta$ degrees.

It may also be noted that these matrices are symmetrical by definition, that is $P(i,j|d,\vartheta) = P(j,i|d,\vartheta)$. This happens because, in calculating the occurrence of the couple $i$ and $j$, whenever it is found in a given spatial arrangement, left-right for example, it is certainly also found in the other direction, that is right-left. In conclusion, if working with G levels of grey, for each distance d at which one wishes to study the texture characteristics, there are four matrices $GxG$ with the above-mentioned properties.

Returning to the assumption that the texture information can be obtained from the SGLD matrices, below are defined the texture features used in the present method of classification of the ROI. To make the SGLD matrices comparable, and therefore also the characteristics extracted, normalisation may be carried out. This normalisation is carried out by imposing on each matrix the constraint:

$$\sum_{ij} p'(i, j \mid d, \vartheta) = 1, .$$

In other words the sum

$$R = \sum_{ij} P(i, j \mid d, \vartheta)$$

is calculated and the matrix element is reassigned, dividing it by $R$.

Starting from the distributions of probability $p'(i,j|d,\vartheta)$, the averages along $i$ and $j$ are defined:

$$p_x(i) = \sum_{j=1}^{N_g} p'(i, j)$$

and

$$p_y(j) = \sum_{i=1}^{N_g} p'(i, j)$$

where $N_G$ is the number of grey levels of the image.

The following may therefore be obtained:

$$\mu_x = \sum_{i=1}^{N_g} i \cdot p_x(i) \qquad\qquad AverageX$$

$$\mu_y = \sum_{j=1}^{N_g} j \cdot p_y(j) \qquad\qquad AverageY$$

$$\sigma_x^2 = \sum_{i=1}^{N_g} (i - \mu_x)^2 p_x(i) \qquad\qquad VarianceX$$

$$\sigma_y^2 = \sum_{j=1}^{N_g} (j - \mu_y)^2 \, p_y(j) \qquad\qquad\qquad VarianceY$$

$$\sum_{i=1}^{N_g} \sum_{j=1}^{N_g} [p'(i,j)]^2 \qquad\qquad\qquad Energy$$

$$\sum_{i=1}^{N_g} \sum_{j=1}^{N_g} (i - \mu_x)(j - \mu_y) p'(i,j) / (\sigma_x \sigma_y) \qquad\qquad Correlation$$

$$\sum_{i=1}^{N_g} \sum_{j=1}^{N_g} (i - j)^2 \, p'(i,j) \qquad\qquad\qquad Inertia$$

$$-\sum_{i=1}^{N_g} \sum_{j=1}^{N_g} p'(i,j) \log_2 p'(i,j) \qquad\qquad\qquad Entropy$$

$$\sum_{i=1}^{N_g} \sum_{j=1}^{N_g} \frac{p'(i,j)}{1 + (i-j)^2} \qquad\qquad Inverse\ difference\ moment$$

Then, defining:

$$p_{x+y}(k) = \sum_{i=1}^{N_g} \sum_{j=1}^{N_g} p'(i,j), \ i + j = k, \ k=2,\dots,2 \cdot N_g$$

we have::

$$SA = \sum_{k=2}^{2N_g} k \cdot p_{x+y}(k) \qquad\qquad\qquad Sum\ of\ the\ average$$

$$\sum_{k=2}^{2N_g} (k - SA)^2 \, p_{x+y}(k) \qquad\qquad\qquad Sum\ of\ the\ variance$$

$$-\sum_{k=2}^{2N_g} p_{x+y}(k) \log_2 p_{x+y}(k) \qquad\qquad\qquad Sum\ of\ the\ entropy$$

In the same way with

$$p_{x-y}(k) = \sum_{i=1}^{N_g}\sum_{j=1}^{N_g} p'(i,j), |i-j|=k, \ k=0,1,2,\ldots,N_g-1$$

we obtain:

$$DA = \sum_{k=0}^{N_g-1} k \cdot p_{x-y}(k) \qquad\qquad \texttt{Difference of the average}$$

$$\sum_{k=0}^{N_g-1}(k-DA)^2 p_{x-y}(k) \qquad\qquad \texttt{Difference of the variance}$$

$$-\sum_{k=0}^{N_g-1} p_{x-y}(k)\log_2 p_{x-y}(k) \qquad\qquad \texttt{Difference of the entropy}$$

$$(Entropia - H_1)/\max\{H_x, H_y\} \qquad \textit{Measure of correlation 1}$$

where

$$H_1 = -\sum_{i=1}^{N_g}\sum_{j=1}^{N_g} p'(i,j)\log_2[p_x(i)p_y(j)],$$

$$H_x = -\sum_{i=1}^{N_g} p_x(i)\log_2 p_x(i)$$

and

$$H_y = -\sum_{j=1}^{N_g} p_y(j)\log_2 p_y(j)$$

$$\sqrt{1 - \exp[-2(H_2 - Entropia]} \qquad \textit{Measure of correlation 2}$$

where

$$H_2 = -\sum_{i=1}^{N_g}\sum_{j=1}^{N_g} p_x(i)p_y(j)\log_2[p_x(i)p_y(j)].$$

These are the 17 features, which are extracted from the SGLD matrices. Processing these matrices at a fixed distance $d$ means extrapolating characteristics, which are correlated with properties that are occured within the image at an interval of $d$ pixels. It was decided to study the ROI with variable values of d ranging from 50 μm to 1 mm, so as to understand both the analysis of the individual microcalcifications and that of the clusters that they compose. In this way, a number of texture characteristics of several hundreds (about 700) are extracted for each ROI. To determine which of these are really significant for the purpose of classification, it is necessary to study them and select them according to the method illustrated in figure 23 and described here below.

The step of selecting the characteristics is based on the measurement of their discriminatory capacity.

It can help us to reject those characteristics that are not at all useful for the purpose of differentiation. For this purpose it is chosen to use the Student test; applying the standard error concept, this test provides us with a measure of the significance of the difference of the means of the two distributions. In particular, we are interested in checking whether the trend of the two distributions around the mean differs systematically or not.

In the simplest cases, distributions with the same variance are estimated; in the present method it is not known how the features behave in malignant and benign cases, while these are the very distributions of which we want to assess the discriminatory capacity, that is the significance of the difference between the means. If we suppose that the variances are not the same, we can study the distributions of the characteristics in the malignant and benign cases, calculating the average and variance for each of them on the basis of all the training cases that we have at our disposal.

The Student distribution, known as $P(t|\gamma)$, is the probability, for $\gamma$ degrees of freedom that a given statistic $t$ may be smaller than that observed if the means were in fact the same.

That is, it may be said that two means are significantly different if, for example, $P(t|\gamma)>0.9$.

In other words, 1- $P(t|\gamma)$ represents the level of significance $\rho$ at which it is decided to reject the hypothesis that the two means are the same. If the Student parameter t is calculated for all the features, a first selection may be made based on the level of significance.

Varying the level of significance is the equivalent of selecting a greater or smaller number of characteristics.

At this point, it is proposed to estimate the linear correlation existing between the characteristics that have survived this first selection phase.

The value of the linear correlation coefficient $r$ varies between *-1* and *1,* indicating, respectively, inverse or direct proportionality. When it is known that a linear correlation exists, $r$ constitutes a conventional method for assessing its force. What we want to do now is define classes of features with a high correlation, so that all the features belonging to the same class have their linear correlation value greater than a fixed threshold, depending on the level of significance.

Grouping the characteristics in order, the procedure is as follows:

- the first group is defined, to which the first feature belongs;
- the linear correlation between the feature being examined and each of the characteristics already analysed is calculated; if the value does not exceed the threshold, this characteristic forms a new group, otherwise it is associated with the group to which belongs the characteristic with which it has the highest coefficient of correlation;
- the procedure continues in this way until the characteristics are exhausted.

Performing a number of tests on the value of the level of significance, an optimum value of 0.1 was established for the preferred configuration. What remains to be done is to determine a criterion for selecting from each group the characteristic most able to represent the properties of the group to which it belongs.

The choice made in the present invention was to select the group element with highest Student $t$ value. In this way the most representative element is extracted from each group.

These characteristics may now be used as input values for a

SVM classifier realised as described previously.

In a possible embodiment of the present invention it is possible to use a genetic algorithm to optimise the choice of the parameters present in the detection and false-positive reduction phases (figures 1, 2). In particular, for the purposes of this optimisation, the parameters regarding the shape and dimensions of the various filters used during detection may be considered, the value of the thresholds for the thresholding phases, the Gaussianity and hard thresholding tests on the wavelet coefficients, the type of wavelet used in the multiresolution analysis, the type of kernel, the value of $C^+$ and C⁻ used in the SVM classifier.

Moreover, it is also possible to use a genetic algorithm in the phase of classifying regions of interest according to their degree of malignity to optimise the choice of the dimensions of the boxes used in pre-processing, the number d of distances in the phase of extraction of characteristics, the levels of significance in the selection of the characteristics, the type of kernel, the value of $C^+$ and $C^-$ used in the SVM classifier.

The genetic algorithm uses analyses individuals composed of different genes; each of these genes represents one of the above-mentioned parameters. The aim, in the detection phase, is to choose the combination which gives the best compromise between the number of true clusters and the number of false-positives per image, while in the phase of classification according to malignity, it is to find the best result in terms of "sensitivity" and "specificity".

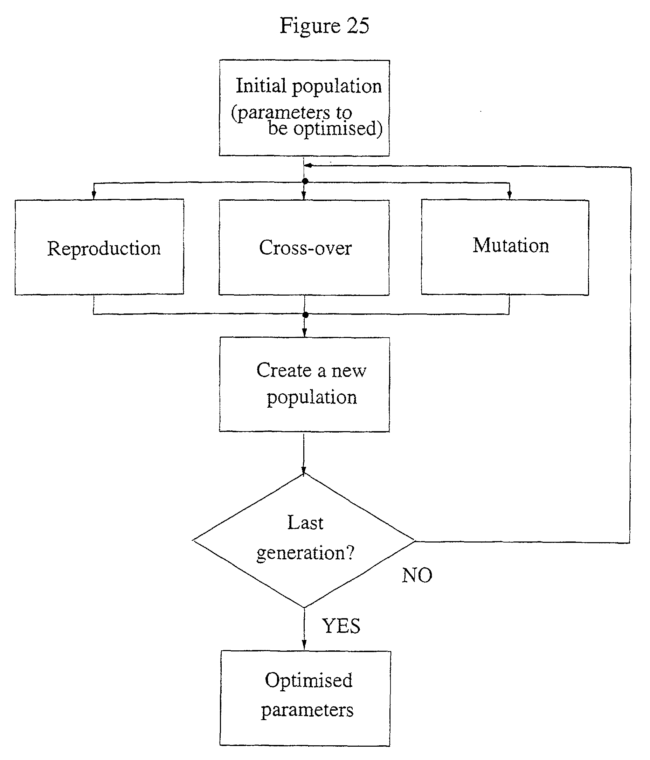

The genetic algorithm implemented is shown in figure 25 and is directly inspired by what happens in nature where, comparing one generation with the next, various types of individuals are found:

- individuals born from the mating of parents present in the previous generation;
- individuals of the previous generation whose genetic heritage is unchanged:
- individuals of the previous generation whose genetic heritage has been changed by random mutations.

The realisation of this type of general substitution is based on an application of genetic operations to the individuals of the initial population; these operations are reproduction, cross-over and mutation. They are not performed one immediately after the other, but different groups of new-born individuals are created, each one of which contains elements obtained from the application of one of the genetic operations.

There will therefore be:

- individuals obtained by cross-over;
- individuals reproduced directly;
- individuals obtained by mutation.

As regards the criterion with which to interrupt the evolution of the population, various possibilities were considered, dwelling particularly in the analysis of the strategies which do not involve a predetermined fixed number of generations. This choice was dictated above all by the poor knowledge of the trend of the fitness functions studied in the case of detection on a mammogram. It was therefore decided that the best decision was to have the genetic algorithm itself show, performing various evolutions, how many generations could be necessary for an analysis of the space subject to research.

Although the previous discussion has been centred essentially on the illustration of a method for the automatic detection of clusters of microcalcifications in a digital signal representing at least one image of at least one portion of mammary tissue, it remains understood that the present invention also comprises any apparatus suited for implementing the method described above.

**Claims**

1. Method for the automatic detection of microcalcifications in a digital signal representing at least one portion of mammary tissue; method comprising the following steps:

   (a) detecting at least one potential microcalcification in said digital signal;
   (b) calculating a set of characteristics for said at least one potential microcalcification;
   (c) eliminating, or not eliminating, said at least one potential microcalcification using a Support Vector Machine classifier (SVM), on the basis of the characteristics calculated.

2. Method according to claim 1, **characterised in that** it is suited for identifying clusters of microcalcifications not eliminated in phase (c) and suited for storing and indicating the position and the extent of said clusters.

3. Method according to either of the previous claims, wherein, in phase (c), said classifier (SVM) weighs differently the errors of a false-negative type and of a false-positive type ($C^+$, $C^-$).

4. Method according to any of the previous claims, wherein, in phase (c), a "boot-strap" learning strategy is used for said classifier (SVM).

5. Method according to any of the claims 2-4, wherein said clusters of microcalcifications are classified according to their degree of malignity using texture characteristics of the digital signals.

6. Method according to claim 5, wherein said classifier (SVM) is used to classify said clusters according to their degree of malignity.

7. Method according to any of the previous claims, wherein a genetic algorithm is used to optimise the choice of the parameters used in phases (a), (b) and (c).

8. Method according to any of the claims 2-7, wherein, in said storage phase, a screen table is used as an instrument to show and/or store regions of interest present in the digital signals.

9. Method according to claim 8, wherein said regions of interest shown by means of the screen table are used to perform training of said classifier (SVM).

10. Method according to claim 8, wherein said regions of interest shown by means of the screen table are classified according to their degree of malignity using texture characteristics of the digital signals.

11. Method according to any of the previous claims, suited for being implemented in an apparatus for processing and analysis of mammographic images.

12. Apparatus suited for implementing a method according to claims 1-10

Figure 1

# Figure 2

Figure 3

## Figure 4

Digital image

↓

Identify
peaks 1) and 4)
figure 3

↓

Subdivide image
in windows

↓

Mark windows
as 0 or 1

↓

Eliminate
islands 0
within tissue 1

↓

Discards regions 1
with small area

↓

Select
roundest area

↓

Check
convexity
on regions 1

↓

Select convex
region with
largest area

↓

Calculate outline
on the original
image

↓

Cropped image

## Figure 5

Cropped image

↓

Noise
equalisation

↓

Linear
filter

↓

Gaussianity
test

↓

Local
thresholding

↓

Signals
extraction

↓

To the next phase
(false positive reduction or OR)

# Figure 6

# Figure 7

## Figure 8

| 0.75 | 0.75 | 0.75 |
|------|------|------|
| 0.75 | 1.0  | 0.75 |
| 0.75 | 0.75 | 0.75 |

## Figure 9

| 1 | 1 | 1 | 1 | 1 | 1 | 1 |
|---|---|---|---|---|---|---|
| 1 | 1 | 1 | 1 | 1 | 1 | 1 |
| 1 | 1 | 0 | 0 | 0 | 1 | 1 |
| 1 | 1 | 0 | 0 | 0 | 1 | 1 |
| 1 | 1 | 0 | 0 | 0 | 1 | 1 |
| 1 | 1 | 1 | 1 | 1 | 1 | 1 |
| 1 | 1 | 1 | 1 | 1 | 1 | 1 |

## Figure 10

# Figure 11

ROI centred on a microcalcification

Fitting surface of the background of the ROI

ROI with corrected background

Figure 12

| Feature type | Description of the feature |
|---|---|
| Compactness | $C=p^2/a$ where $p$ is the perimeter and $a$ the area |
| Area | Number of pixels that compose the object |
| Mean grey level | Mean of the grey levels of the pixels in the object |
| Grey level | Root-mean square (RMS) of the fluctuations of the grey level in the microcalcification |
| Shape | RMS of the distance from the centre of the pixels on the perimeter |
| Dimensions | Horizontal and vertical length of the signal |
| 'Holes' in microcalcifications | Area of the hole in the centre of the microcalcifications |
| Contrast | Difference between the central area and the mean in a 3x3 neighbourhood |
| Compactness | Ratio between the number of objects in the background and number of pixels in the microcalcification |
| Area describer | Standard deviation of the ratio between the area of the microcalcification and the mean area |
| Local background | RMS of the noise fluctuations in a $N x N$ window |
| Local background | Mean grey level of the local background |
| Contrast | Difference between the maximum intensity in the calcification and the mean intensity of the local background |
| Contrast | Ratio between the mean grey level of the microcalcification and the mean intensity of the local background |
| Contrast | Difference between the mean grey level of the microcalcification and the mean intensity of the local background |
| Shape | Ratio between the area of the microcalcification and the square of the maximum linear dimension |
| Shape | $p/4\pi a$ where $p$ id the perimeter and $a$ the area |
| Edge gradient | Mean value of the Robert gradient for each pixel on the perimeter |
| Edge gradient | Mean value of the Sobel gradient for each pixel on the perimeter |
| Contrast | Intensity of the central pixel minus the mean values of the pixels on the perimeter, all divided by the RMS of the noise fluctuations in the object |
| Shape | Ratio between maximum radius and minimum radius of the microcalcification |
| Contrast | Mean grey level of the microcalcification minus mean value of the 2x2 neighbourhood |
| Degree of linearity | Measurement of the extension of the microcalcification along a privileged direction |
| Average local gradient | Value of the local gradient linked to the degree of linearity |

## Figure 13

## Figure 14

Small and non representative training set

SVM training on the training set

SVM test on new examples

Collecting incorrectly classified signals

Integration of these signals in the training set

N. Bootstrap iterations terminated ?

NO

YES

Training terminated

# Figure 15

Cropped image

↓

Extraction of
a window

↓

Preliminary
filter

↓

Histogram analysis
and thresholding

↓

Is the window
a ROI?  — NO

↓ YES

Wavelet
filter

↓

Histogram analysis
and thresholding

↓

Is the window
a ROI?  — NO

↓ YES

Store objects
found

↓

Image
analysis
completed? — NO

↓ YES

To the next phase
(false positive reduction
or OR)

# Figure 16

Original
signal

$A_1$

$A_2$

$A_3$

$D_1$

$D_2$

$D_3$

# Figure 17

# Figure 18

a)

b)

c)

d)

Figure 19

Fitting curve

First peak found

Figure 20

## Figure 21

| | Response: (Signal present ?) | |
| | "Yes" | "No" |
|---|---|---|
| Present | HIT *(True-positive)* | MISS *(False-negative)* |
| Absent | FALSE ALARM *(False-positive)* | CORRECT REJECTION *(True-negative)* |

Stimulus

## Figure 22

TPF vs. Number of false-positives per image

# Figure 23

Region of interest (ROI)

Pre-processing

Extract texture characteristics ← Parameters optimisation

Select characteristics

SVM classifier

Classification results

Classify ROI according to degree of malignity

# Figure 24

1

$d_1$

2

$d_2$

(i,j)

4

$d_4$

$d_3$

3

Figure 25